Europäisches Patentamt

European Patent Office

Office européen des brevets

(11) Publication number: **0 235 591**
A1

# (12) EUROPEAN PATENT APPLICATION

(21) Application number: 87101360.3

(22) Date of filing: 02.02.87

(51) Int. Cl.⁴: **A 61 M 1/14, A 61 M 1/34**

(30) Priority: 05.02.86 IT 333086

(43) Date of publication of application: 09.09.87
Bulletin 87/37

(84) Designated Contracting States: **AT BE CH DE ES FR GB LI**

(71) Applicant: **Donatelli, Demetrio, Via Mameli 53, I-41037 Mirandola, Province of Modena (IT)**

(72) Inventor: **Donatelli, Demetrio, Via Mameli 53, I-41037 Mirandola, Province of Modena (IT)**

(74) Representative: **Modiano, Guido et al, MODIANO, JOSIF, PISANTY & STAUB Modiano & Associati Via Meravigli, 16, I-20123 Milan (IT)**

(54) **Device for the automatic actuation of haemodialysis processes without anticoagulants.**

(57) The present invention relates to a device for the automatic actuation of haemodialysis processes in the absence of anticoagulant substances in processed blood, which comprises a modular control unit (1) for the intervention in presettable sequences and times of a plurality of electric pincers (2) for the occlusion and the opening of the passage of blood in a closed circuit (3) of haemodialysis tubing (4). A first one of said electric pincers (2a) is interposed between a tube (4a) leading out from a patient (P) and an aspirating pump (5) acting on the circuit (3). A second pincer (2b) is interposed between a container (6) of physiological solution and a connection (7) for the feeding of said solution into the circuit (3), which connection is arranged upstream of said aspirating pump (5), downstream of which a blood filtering element (8) is provided, the output of which is connected to the patient (P) with the interposition of a third electric pincer (2c).

0235591

DEVICE FOR THE AUTOMATIC ACTUATION OF HAEMODIALYSIS PROCESSES IN THE ABSENCE OF ANTICOAGULANT SUBSTANCES IN PROCESSED BLOOD

The present invention relates to a device for the automatic actuation of haemodialysis processes in the absence of anticoagulant substances in processed blood.

It is known that substances for preventing blood coagulation are introduced in haemodialysis processes, in order to prevent deposits and therefore the clogging of the filtering assembly.

The substance most commonly used for this purpose is heparin, which however, by virtue of its very anticoagulant nature, renders extremely unsuitable, if not impossible, the execution of haemodialysis processes on patients suffering from ulcer or recovering from surgery.

On the other hand, haemodialysis has been attempted without introducing such anticoagulant substances, overcoming the tendency of the filter assembly to clog by means of periodic manual interventions of an operator, consisting in an actual interruption of the haemodialysis and washing of the above-mentioned filter assembly with physiological solutions.

However, besides the above described disadvantages related to the presence of anticoagulant substances, disadvantages have also been found in the second method, mainly due to the incorrect or approximate evaluation of the washing times for the filter, either too long or too short, and, despite these washes, clogging has nevertheless occurred, with a consequent loss of blood for the patient; the need thereby arises to arrange the replacement of the filter assembly and the consequent need for the constant presence of a trained operator.

The technical aim of the present invention is to provide a device for the automatic actuation of haemodialysis processes in the absence of anticoagulant substances in the processed blood, which overcomes the disadvantages of the known art, which operates according to precise times which can be preset by a specialized operator and can, possibly, be modified during the process itself, which furthermore allows one to perform haemodialysis even on patients suffering from ulcer or recovering from surgery and does not require the constant presence of trained personnel.

This aim and other objects which will become apparent hereinafter are achieved by a device for the automatic actuation of haemodialysis processes in the absence of anticoagulant substances in processed blood, characterized in that it comprises a modular control unit for the intervention in presettable sequences and times of a plurality of electric pincers for the occlusion and the opening of the passage of blood in a closed circuit of haemodialysis tubing, at least one first electric pincer interposed between a tube leading out from a patient and an aspirating pump adapted for acting on the circuit, at least one second electric pincer interposed between a container of physiological solution and a connection for the introduction of said solution into the circuit, which connection is arranged upstream of said aspirating pump, downstream of which a blood filtering element is provided, the output of which is connected to the patient with the interposition of at least one third electric pincer.

Further characteristics and advantages of the invention will become apparent from the description of a preferred, but not exclusive, embodiment of a device for the automatic actuation of haemodialysis processes in the absence of anticoagulant substances in the processed blood, illustrated only by way of non-limitative example in the accompanying drawing, wherein:

Fig. 1 is a schematic view of the overall structure of the device according to the invention illustrating the use thereof;

Fig. 2 is a schematic diagram of an intervention cycle of three electric pincers in the course of time.

More in detail, the reference numeral 1 indicates a modular control unit for the intervention of a plurality of electric pincers 2 for the occlusion and the opening of the passage of blood in a closed circuit 3 of tubing 4 for haemodialysis. A first electric pincer 2a is interposed between an element of tubing 4a leading out from a patient P and an aspirating pump 5 acting on the tubing 4; a second electric pincer 2b is interposed between a container 6 of physiological solution or the like and a connection 7 for the introduction thereof into the circuit 3, which connection is arranged upstream of the aspirating pump 5.

Downstream of the pump 5, a filter element 8 is provided for the blood, the output of which is connected to the patient P with the interposition of a third electric pincer 2c; the direction of the flow of blood and of physiological solution is indicated by the arrows A.

The modular unit 1 is composed of a portion 1a for the general actuation of the operation, provided with an on/off switch 1b and the related indicator light 1c, and of a plurality of actuator modules 1d each of which is connected to the portion 1a and controls an electric pincer 2 by means of electric cables 1e and is provided with an adjustment and control potentiometer 1f and with visual indication apparatus such as a pair of indicator lights 1g advantageously of different and contrasting colors.

The operation of the invention is as follows: the blood to be processed is drawn via the tubing 4 from the patient P under the action of the pump 5, is sent through the filter 8, in which it discharges the impurities accumulated in the organism, and is then returned to the patient P.

In this operating cycle, the first electric pincer 2a is open, the second one 2b is closed, the third one 2c is also open.

If the filter is to be washed, according to the cyclic times preferably expressed in minutes and preset by an operator on the potentiometers 1f of the actuator modules 1d, the latter act so that the first electric pincer 2a cuts off the passage of the flow of blood leaving the patient P for an interval of time ranging, e.g., between 30 and 90 seconds, during this interval, and for the entire identical duration thereof, the second electric pincer 2b opens, thus allowing the passage of physiological solution from the container 6 to the pump 5 and, to the filter 8. The third electric pincer 2c, during said time interval, alternately closes and opens, almost in pulses, the passage of the tubing 4 which leads to the patient P from the output of the filter 8.

This induces, inside the same filter 8, a series of overpressure pulses so that the physiological solution contained therein flows with turbulence, with splashing of the liquid mass and a consequently more effective action of removal of any aggregates or material which clogs the pores of the filter mesh.

Once this time interval has expired, the haemodialysis process is restored as described above until it is time for a new cleaning interval.

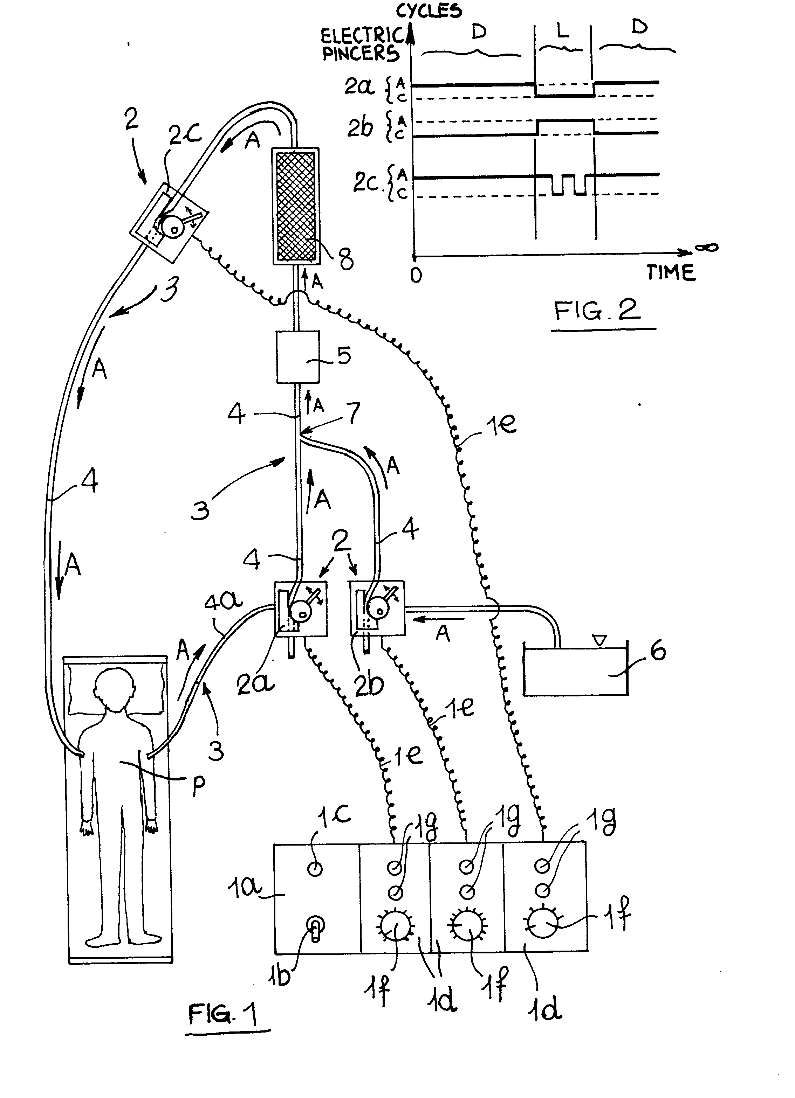

The operative cycles of the electric pincers 2a, 2b, 2c are qualitatively indicated in figure 2, wherein the active cycle of each electric pincer is plotted on the Cartesian cycles/times graph with an unbroken line: the upper diagram relates to the first electric pincer 2a, the centre diagram relates to the second pincer 2b and the lower diagram relates to the third pincer 2c; the letters "A" and "C" respectively indicate the "Open" and "Closed" conditions. Proceeding from the ordinate t=0 towards t=*, the dialysis of the patient occurs in the vertical region "D" of the diagram; the washing of the filter 8 occurs in the region "L"; dialysis occurs again in the region "D", and so on in repetitive cycles.

It should be noted that the modular unit 1 is equipped with a manual reset safety device which can be used if, while performing the dialysis, its functionality is interrupted for accidental reasons.

In practice, it has been observed that the invention as described herein achieves the intended aims, performing a perfect dialysis, maintaining the characteristics of the filter without introducing anticoagulants such as, e.g.,

heparin.

The invention thus conceived is susceptible of numerous modifications and variations, all of which are within the scope of the inventive concept.

Moreover, all the details can be replaced by other technically equivalent elements.

Thus, for example, the electric pincers may be replaced by other tube occlusion or shut-of means, such as valve means.

In practice, the materials employed, as well as the dimensions, may be any according to the requirements, without thereby abandoning the scope of protection of the following claims.

CLAIMS

1. Device for the automatic actuation of haemodialysis processes in the absence of anticoagulant substances in processed blood, characterized in that it comprises a modular control unit for the intervention in presettable sequences and times of a plurality of electric pincers for the occlusion and the opening of the passage of blood in a closed circuit of haemodialysis tubing, at least one first electric pincer interposed between an element of tubing leading out from a patient and an aspirating pump adapted for acting on the circuit, at least one second electric pincer interposed between a container of physiological solution and a connection for the introduction of said solution into the circuit, which connection is arranged upstream of said aspirating pump, downstream of which a blood filtering element is provided, the output of which is connected to the patient with the interposition of at least one electric pincer.

2. Device according to claim 1, characterized in that said modular control unit is composed of a general actuator part for operation, provided with switch means and indicator means, for controlling a number of actuator modules connected thereto corresponding to the number of electric pincers used.

3. Device according to claim 2, characterized in that each of said actuator modules is connected to at least one of said electric pincers by means of electric cables and is provided with a potentiometer for adjusting and controlling the duration of the intervention of the electric pincer.

4. Device according to claim 1, characterized in that said electric pincers have an intervention pattern according to which during the opening times of said first pincer, said second pincer is closed, and vice versa.

5. Device according to claim 2, characterized in that said switch means comprise at least one on/off switch, and in that said indicator means comprise visual indication apparatus.

6. Device according to claim 1 or 4, characterized in that said third electric pincer is adapted for causing pulse-like interventions of the opening of said second pincer.

7. Device according to claim 1 or 2, characterized in that said modular control unit is equipped with a manual reset safety device.

8. Device according to one or more of the preceding claims, characterized in that said first pincer is adapted to be closed for cutting-off the flow of blood leaving the patient, and in that said second pincer is adapted to be successively opened for a time interval for allowing the passage of said physiological fluid from said container to said pump and said blood filtering element.

9. Device according to claim 8, characterized in that said second pincer is adapted for being opened for a time interval ranging from about 30 to 90 seconds.

10. Device according to one or more of the preceding claims, characterized in that said third pincer is adapted to be alternately opened and closed during said time interval for causing overpressure pulses of said physiological solution to flow turbulently into said blood filtering element.

1/1

0235591

FIG. 2

FIG. 1

European Patent Office

**EUROPEAN SEARCH REPORT**

0235591
Application number

| | DOCUMENTS CONSIDERED TO BE RELEVANT | | EP 87101360.3 |

| Category | Citation of document with indication, where appropriate, of relevant passages | Relevant to claim | CLASSIFICATION OF THE APPLICATION (Int Cl 4) |
|---|---|---|---|
| Y | WO – A1 – 83/04 373 (A.RAVET) | 1,2,4, 5,8 | A 61 M 1/14 |
| A | * Totality; especially fig. 1; page 3, line 18 – page 4, line 8; page 4, lines 18-21 * | 3,9 | A 61 M 1/34 |
| | -- | | |
| P,Y | EP – A2 – 0 181 139 (KANEGAFUCHI KAG.)(14-05-1986) | 1,2,4, 5,8 | |
| A | * Totality; especially fig. 2; page 9, lines 20-24; page 25, line 15 – page 26, line 8; page 27, lines 16-25 * | 3,6,7, 9 | |
| | & FI-A-854 371 (07-05-1986) AU-A1-49 083/85 (15-05-1986) JP-A2-61-113 457 (31-05-1986) | | |
| | -- | | |
| A | EP – A1 – 0 148 319 (INTERMEDICAT) | 1 | |
| | * Totality * | | TECHNICAL FIELDS SEARCHED (Int Cl 4) |
| | -- | | |
| A | US – A – 4 334 988 (L.J.MILLIGAN) | 1 | A 61 M 1/00 |
| | * Totality; especially fig. 2; column6, lines 29-64 * | | |
| | -- | | |
| A | EP – A2 – 0 093 542 (KURARAY CO.) | | |
| | * Totality; especially fig. 1,6; abstract * | | |
| | ---- | | |

The present search report has been drawn up for all claims

| Place of search | Date of completion of the search | Examiner |
|---|---|---|
| VIENNA | 20-05-1987 | LUDWIG |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82